# EUROPEAN PATENT SPECIFICATION

(11) **EP 0 504 538 B1**
(45) Date of publication and mention of the grant of the patent: **16.10.1996**
(21) Application number: 91830097.1
(22) Date of filing: 12.03.1991
(51) Int. Cl.: A61M 5/00, A61M 5/32, G07F 7/06

(54) **Apparatus for the collection of used syringes and the conditional distribution of new syringes**
Vorrichtung zur Aufnahme gebrauchter Spritzen und gleichzeitigen Ausgabe neuer Spritzen
Appareil pour la récupération de seringues usagées et la distribution conditionnelle de nouvelles seringues

(43) Date of publication of application: 23.09.1992
(73) Proprietor: IAZOMATIC S.r.l., I-04011 Aprilia LT (IT)
(72) Inventor: Ianni, Salvatore, I-00139 Roma RM (IT)
(74) Representative: Bazzichelli, Alfredo

(56) References cited:
- WO-A-89/09982
- DE-U- 8 520 184
- NL-A- 8 900 310
- US-A- 3 794 148
- US-A- 4 869 366
- US-A- 4 919 264

## Description

The present invention refers to an apparatus for the collection of used syringes and the distribution of new syringes, in which said distribution of each new syringe is dependent upon the introduction into said apparatus of a used syringe, and is further conditioned by the presence of the needle in the latter.

A serious problem afflicting modern society derives from the dangerous presence of used syringes abandoned by drug addicts in the widest variety of places, and in particular in parks and fields frequented mainly by children.

Another problem, relating to the one described above, derives from the difficulties regarding the distribution free of charge of new syringes to the individuals mentioned above, as neither the state health organizations, nor chemists, are able or willing to take on the burden of said service, due to obvious reasons of further employment of personnel, expense and, last but not least, danger.

The object of the present invention is to solve the problems mentioned above by providing an apparatus capable of accepting used syringes and distributing in exchange new syringes, as long as said used syringes being introduced into the apparatus in question are complete with their respective needles, this in order to force the user not to neglect the elimination of the needles, which form the source of the danger of infection.

WO-A-8 909 982 discloses an automat for one time-syringes capable of dispensing new syringes as reaction to the insertion of used syringes through the use of a comparing arrangement consisting of a photocell in a recording box. On the contrary, the present invention makes use of a comparing arrangement with mechanical working with the condition that a new syringe is released only if on the used one the needle is still present.

An adequate number of apparatuses according to the present invention installed in appropriate places would mean, consequently, that the collection of used and dangerous syringes would be carried out by the users themselves, as it would be in their own interest to exchange free of charge and without problems a used syringe for a new one, thus substantially resolving the problems relating to the collection services in use at the present time and which, at least up to date, have proved themselves to be insufficient and expensive.

Last, but not least, the use of the same syringe by more than one person would be avoided, thus preventing the spread of infectious diseases, such as AIDS, in this manner.

Therefore the present invention refers to an apparatus for the collection of used syringes 10 and the conditional distribution of new syringes 21 comprising:
a) a cupboard-shaped container 1 having at least one door provided with a safety lock providing access to the inside, said container being divided by a dividing wall 2 parallel to the side walls into a first compartment 3 and a second compartment 4 destined respectively for the collection of said used syringes and the distribution of said new syringes;
b) a first and a second opening in said container respectively for the insertion of used syringes into said first compartment and the delivery of new syringes from said second compartment;
c) an acceptor roller 7 rotatably supported inside said first compartment on shaft 11 said roller being circumferentially divided into a number of axially extending housings each one of said housings being capable of housing a used syringe inserted through said first opening in said container, said roller being formed by a cylinder 8 fixed to said shaft, a first end of said shaft being rotatably supported on said container while the other end being rotatably supported on a plate 12;
d) manual activating means 14 to make said acceptor roller rotate by single steps, so as to bring each one of said housings successively into line with said first opening;
e) pushing means 17 cooperating with said activating means to slide said used syringes introduced into one of said housings on said acceptor roller and aligned to a calibrated bore placed at the end of a notch 15 obtained in said plate;
f) a tank 18 situated below said acceptor roller to collect the used syringes that fall therein by gravity at said single steps in said acceptor roller move around;
g) a circular toothed conveyor belt 19 situated in said second compartment, perimetrally showing a succession of receptacles, each one of which contains a respecting new syringe the teeth 22 of said conveyor belt being engaged with a first pair of idle gear wheels 23 and a second pair of driving gear wheels 24 provided with activated means capable of rotating them in single sections so as to bring in succession each of said receptacles into correspondence with said second opening in said container for the removal of the new syringe within it;
h) a blocking system to block the rotation of said conveyor belt, that can be un-blocked only by the protrusion of the needle of said used syringe through said calibrated bore;
i) means to bring said blocking system for the rotation of said conveyor belt back into an operative position following the rotation by one step of said conveyor belt, and to cause said used syringe to return to its starting position in its housing on said acceptor roller,
characterized by the fact that
said pushing means may slide said used syringe only if this is provided with its needle, thereby causing said conveyor belt being unlocked by said needle and letting the apparatus release a new syringe.

The present invention will be better illustrated here below in the description of one of its preferred embodiments, given as a non-limiting example, with reference to the enclosed drawings, in which:

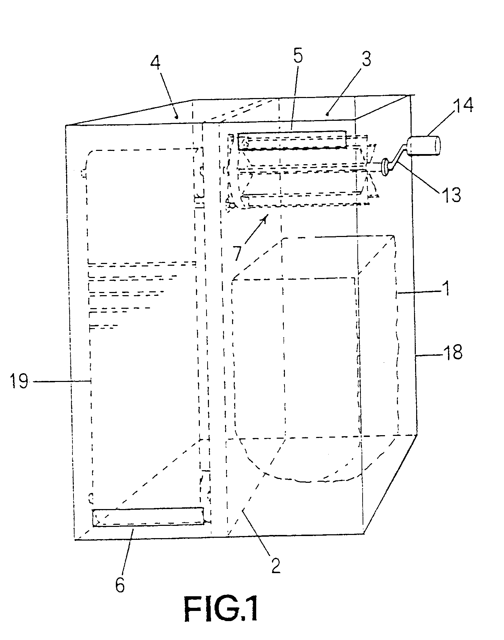

figure 1 is a diagrammatic view in perspective of the subject apparatus; and
figure 2 is a diagrammatic view in longitudinal cross-section, shown in an enlarged scale, and with certain parts removed, of the apparatus according to figure 1.

With reference to the figures of the drawings, with 1 is indicated a cupboard-shaped container having the form of a parallelepiped and constructed in suitably rigid and resistant material, for example metal plate or the like, provided with one or two doors (not shown), with safety locks, through which to access the interior.

The internal space within container 1 is divided by a central longitudinal wall 2 into two compartments, indicated with 3 and 4, intended for the collection of the used syringes and the distribution of the new syringes, respectively. For said collection and distribution, there are provided on the front panel of the container 1 a first upper opening 5 and a second lower opening 6, in communication with compartments 3 and 4, respectively.

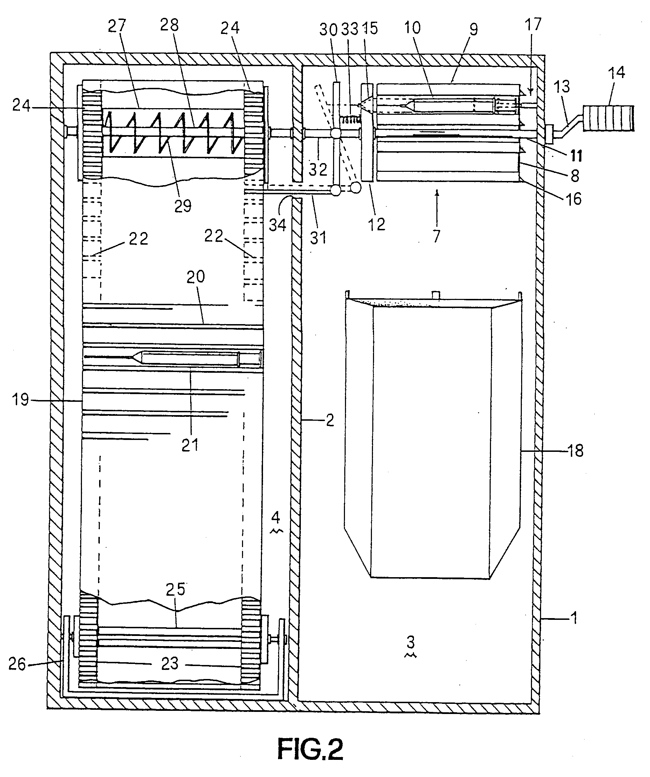

Taking into examination firstly compartment 3, predisposed for collection of the used syringes, as canbe more clearly seen in figure 2, an acceptor roller, generically indicated with 7, is mounted in the upper part of said compartment and is formed by a cylinder 8 integral with a number of radial tongues 9, circumferentially equidistant in such a way as to form between each adjacent pair of said tongues a housing suitable to receive a used syringe 10.

The cylinder 8 is fixed to a shaft 11, a first end of which is rotatably supported, by means of a bearing, on the right side wall (as can be seen in the figure) of the container 1, said first end extending outward from said right side wall for a short distance, whereas the other end of the shaft is rotatably supported, by means of a bearing, on a plate 12 fixed to the inner surface of the rear wall of the container 1, said plate being parallel to said right side wall of the latter.

On said piece of the first end of shaft 11 extending outward from container 1 is fixed a crank 13, equipped with a handle 14, controlling the rotation of the acceptor roller 7 by single steps such as to present at successive intervals each of said housings in said acceptor roller in correspondence with the opening 5 in the front wall of the container 1.

In the thickness of the support plate 12 is formed a notch 15 of a shape similar to that of the front end of the syringe 10. At the end of said notch is formed a calibrated bore of a diameter substantially equal to that of the needle of the syringe 10.

On the end of each tongue 9 adjacent to the right side wall of the container 1, there is a projection 16 made in such a way as to charge a hammer, schematically illustrated and generally indicated with 17, causing it to retreat, following the reciprocal engagement during the rotation of the acceptor roller 7, into a tubular guide fixed to the inner surface of said side wall of the container 1, with the simultaneous compression of a helicoidal spring lying behind, said hammer 17 being arranged in such a manner as to be centered on the bottom of the syringe 10 when the latter, placed in its housing on the acceptor roller 7, is aligned with said notch 15 in the support plate 12.

Below the acceptor roller 7 is movably fixed a tank 18 for collection of the used syringes.

Passing now to consider the compartment 4 for the distribution of new syringes, with 19 is indicated an endless conveyor belt on the outer surface of which are formed in succession, by means of externally protruding equidistant tongues 20, a number of receptacles, each one of which containing a respective new syringe, of which one only is shown and indicated by 21.

The conveyor belt 19 has the inner edges provided with external equidistant teeth 22 engaged with respective lower idle gear wheels 23 and respective upper driving gear wheels 24, the former wheels being integrally connected by an axle 25, rotatably supported by means of bearings on the vertical wings of a bracket 26, while the latter wheels are integrally connected by a drum 27 fixed to a shaft 28, rotatably supported by means of bearings by the left side wall of the container 1 and by the central dividing wall 2, as shown in the figures, said drum 27 being activated by a helical spring 29, suitably pre-charged and rechargeable, capable of causing said drum to rotate in a clockwise direction one step at a time, each of said steps bringing one of said receptacles into correspondence with the lower opening 6 in the front wall of the container 1. Between the mechanism for acceptance of used syringes in the compartment 3 of the container 1 and the mechanism for distribution of new syringes in the compartment 4, described above, there is provided a release device which allows the activation of the latter only if the used syringe introduced into the former is complete with its relative needle.

Said release device is formed by a system of articulated levers comprising a first and a second lever 30 and 31 respectively, hinged together.

The first lever 30 is supported at its central point on a horizontal support bar 32, the ends of which are fixed to the plate 12 and to the dividing wall 2, respectively, said first lever being normally maintained in a vertical position opposite the plate 12 by means of a helical spring 33 connecting it to said plate, said position corresponding to the blocked condition of said mechanism for the distribution of new syringes.

The second lever 31 has one end supported on the lower end of the first lever 30, and extends horizontally through a supporting and guiding bore 34 formed in the dividing wall 2 until keying with its free extremity a tooth 22 on the corresponding internal side edge of the conveyor belt 19.

When in function, after having introduced a used syringe, complete with its needle, through the upper opening 5 in the front wall of the container 1, so as to place said syringe, in the position illustrated in figure 2, in the housing between two radial tongues 9 of the acceptor roller 7 situated in correspondence with said opening, the acceptor roller 7 is made to rotate one step in a clockwise direction by means of the crank 13 and relative handle 14. During said rotation the projection 16, situated on the tongue 9 forming the lower surface of said housing, when engaging with the hammer 17 will cause the loading of the latter, causing it to recede into said tubular guide, with the consequent compression of the relative spring placed behind, so that following the disengaging of said tongue from said hammer as said rotation proceeds, said hammer will leap forwards, hitting the base of the syringe situated in said housing in such a way as to thrust it into said housing until the head of said syringe is inserted in the notch 15, causing the needle to come out through said calibrated through bore in the base. Said needle will violently bump into the upper part of the first lever 30, forcing said upper part to rotate in a clockwise direction around its fulcrum on the support bar 32, causing the consequent movement of the lower end, hinged to the second lever 31, in such a way to remove the free end of said second lever 31 from its engagement with the tooth of the conveyor belt 19, so as to allow the rotation of said belt. The positions of said first and said second lever 30 and 31 are determined by the emerging of the needle of the used syringe 10, and are shown with dotted lines in figure 2.

Following said unlocking of the conveyor belt 19 the helical spring 29 in the drum 27 connecting the driving gear wheels 24 will cause both wheels to perform a clockwise rotation of one step, thus pulling in said direction the conveyor belt 19, so as to bring one of its receptacles with its relative new syringe contained therein into line with the lower opening 6 in the front wall of the container 1, to allow the removal of the syringe itself.

Immediately after said unlocking of the conveyor belt 19 and its advancement by one step, the spring 33, stressed by said rotation of the first lever 30, will cause the latter to return to a vertical position, causing the consequent movement of the second lever 31 into the position blocking said distribution mechanism. Said return of said first lever 30 performs at the same time a pushing action on the needle sticking out of said calibrated bore, such as to return the associated used syringe into its starting position within its housing in the acceptor roller 7, to fall later into the collection tank 18 when said housing, after successive rotations of the acceptor roller 7, finds itself in correspondence with said tank.

It must here be noted that the new syringes contained in said housings on the conveyor belt 19 can eventually be manufactured complete with respective phials of physiological solution or distilled water.

It must also be noted that, alternatively, the charging of the spring 28 for the rotation of the drum 27 and the relative driving gear wheels 24 on the conveyor belt 19 can take place following the rotation of the acceptor roller 7, by suitably connecting the respective shafts 8 and 28.

The present invention is not limited to the embodiment described herein, but comprises any variants thereof.

## Claims

1. Apparatus for the collection of used syringes (10) and the conditional distribution of new syringes (21) comprising:
a) a cupboard-shaped container (1) having at least one door provided with a safety lock providing access to the inside, said container being divided by a dividing wall (2) parallel to the side walls into a first compartment (3) and a second compartment (4) destined respectively for the collection of said used syringes and the distribution of said new syringes; b) a first and a second opening in said container respectively for the insertion of used syringes into said first compartment and the delivery of new syringes from said second compartment;
c) an acceptor roller (7) rotatably supported inside said first compartment on shaft (11) said roller being circumferentially divided into a number of axially extending housings each one of said housings being capable of housing a used syringe inserted through said first opening in said container, said roller being formed by a cylinder (8) fixed to said shaft, a first end of said shaft being rotatably supported on said container while the other end being rotatably supported on a plate (12);
d) manual activating means (14) to make said acceptor roller rotate by single steps, so as to bring each one of said housings successively into line with said first opening;
e) pushing means (17) cooperating with said activating means to slide said used syringes introduced into one of said housings on said acceptor roller and aligned to a calibrated bore placed at the end of a notch (15) obtained in said plate;
f) a tank (18) situated below said acceptor roller to collect the used syringes that fall therein by gravity at said single steps in said acceptor roller move around;
g) a circular toothed conveyor belt (19) situated in said second compartment, perimetrally showing a succession of receptacles, each one of which contains a respecting new syringe the teeth (22) of said conveyor belt being engaged with a first pair of idle gear wheels (23) and a second pair of driving gear wheels (24) provided with activated means capable of rotating them in single sections so as to bring in succession each of said receptacles into correspondence with said second opening in said container for the removal of the new syringe within it;
h) a blocking system to block the rotation of said conveyor belt, that can be un-blocked only by the protrusion of the needle of said used syringe through said calibrated bore;
i) means to bring said blocking system for the rotation of said conveyor belt back into an operative position following the rotation by one step of said conveyor belt, and to cause said used syringe to return to its starting position in its housing on said acceptor roller,
characterized by the fact that
said pushing means may slide said used syringe only if this is provided with its needle, thereby causing said conveyor belt being unlocked by said needle and letting the apparatus release a new syringe.

2. Apparatus according to claim 1, in which said acceptor roller is formed by a cylinder integral with numerous radial tongues (9), circumferentially equidistant one from the other, in such a way as to form between each adjacent pair of said tongues one of said housings.

3. Apparatus according to claims 1 and 2, in which said manual activation means to make said acceptor roller rotate by single steps are formed by a crank (13) situated outside said container, capable of controlling the rotation by single steps of said acceptor roller.

4. Apparatus according to any one of claims 1 to 3, in which said pushing means are formed by a spring hammer (17) which, loaded by passing over a projection (16), situated on the corresponding side of each of said tongues on said acceptor roller, during the rotation of the latter, springs forwards when no longer engaged with said projection as said acceptor roller rotates, so as to violently hit the base of said used syringe situated in correspondence with it.

5. Apparatus according to any one of the preceding claims, in which said activation means for the rotation by single steps of said pair of gear driving wheels are formed by a helical spring (29) wrapped around a shaft (28) connecting said gear driving wheels.

6. Apparatus according to any one of the preceding claims, in which said blocking system of the rotation of said conveyor belt comprises a first lever (30) hinged at its mid-point and normally kept, by means of a helical spring (33), in a vertical position with its upper part opposite said calibrated bore, said calibrated bore being intended for the protruding of the needle of a used syringe collected in said acceptor roller, and a second lever (31) with one end hinged to the lower end of said first lever and which extends horizontally until engaging with one tooth of said conveyor belt, so as to prevent the rotation thereof, the arrangement being such that when the needle of the above mentioned used syringe protrudes from said calibrated bore it violently hits against said upper part of said first lever, thanks to the impact force gained when said used syringe slides in its housing in said acceptor roller following the action of said spring hammer, causing a rotation in a clockwise direction of the latter around said hinge at its mid-point, with the consequent drawing of said second lever out of engagement with said tooth of said conveyor belt, which is thus free to rotate.

7. Apparatus according to any one of the preceding claims, in which said means for returning said system for blocking the rotation of said conveyor belt into an operative position and for returning said used syringe into its starting position within the housing on said acceptor roller, are formed by said helical spring, destined normally to keep said first lever of said system for blocking the rotation of said conveyor belt in a vertical position, said spring, stressed by the clockwise rotation of said first lever determined by the protruding of said needle through said calibrated bore, causes said first lever to counter-rotate rapidly in an anti-clockwise direction, bringing it back into said vertical position, with a consequent movement of said second lever engaged with one tooth of said conveyor belt, said first lever exerting during said anti-clockwise rotation a pushing action on said needle protruding from said calibrated bore, so as to cause the used syringe associated to said needle to slide back until reaching the position which it originally had within its housing on said acceptor roller before undergoing the thrust of said spring hammer.

8. Apparatus according to any one of the preceding claims, in which said new syringes within said receptacles on said conveyor belt are packed complete with a phial of physiological solution or of distilled water.

## Patentansprüche

1. Vorrichtung zum Sammeln benutzter Spritzen (10) und zur bedingten Abgabe neuer Spritzen (21), die umfaßt:
a) einen schrankförmigen Behälter (1) mit wenigstens einer Tür, die mit einem Sicherheitsschloß versehen ist und Zugang zum Inneren ermöglicht, wobei der Behälter durch eine Trennwand (2) parallel zu den Seitenwänden in eine erste Kammer (3) und eine zweite Kammer (4) unterteilt ist, die zum Sammeln der benutzten Spritzen bzw. zum Abgeben der neuen Spritzen bestimmt sind;
b) eine erste und eine zweite Öffnung in dem Behälter zum Einführen benutzter Spritzen in die erste Kammer bzw. zum Abgeben neuer Spritzen aus der zweiten Kammer;
c) eine Aufnahmerolle (7), die im Inneren der ersten Kammer auf einer Welle (11) drehbar gelagert ist, wobei die Rolle in Umfangsrichtung in eine Reihe sich axial erstreckender Gehäuse unterteilt ist, wobei jedes der Gehäuse eine gebrauchte Spritze aufnehmen kann, die durch die erste Öffnung in dem Behälter eingeführt wird, wobei die Rolle durch einen auf der Welle befestigten Zylinder (8) gebildet wird, wobei ein erstes Ende der Welle drehbar an dem Behälter gelagert ist, während das andere Ende drehbar an einer Platte (12) gelagert ist;
d) eine manuelle Betätigungseinrichtung (14), mit der die Aufnahmerolle in einzelnen Schritten gedreht wird, um jedes der Gehäuse nacheinander auf die erste Öffnung auszurichten;
e) eine Drückeinrichtung (17), die mit der Betätigungseinrichtung zusammenwirkt und gebrauchte Spritzen, die in eines der Gehäuse an der Aufnahmerolle eingeführt und auf eine Maßbohrung am Ende einer in der Platte ausgebildeten Aussparung (15) ausgerichtet werden, verschiebt;
f) ein Gefäß (18), das unterhalb der Aufnahmerolle angeordnet ist und die gebrauchten Spritzen sammelt, die durch die Schwerkraft bei den einzelnen Schritten, in denen sich die Aufnahmerolle herumbewegt, darin hineinfallen;
g) ein kreisförmiges, mit Zähnen versehenes Förderband (19), das sich in der zweiten Kammer befindet und am Umfang eine Abfolge von Aufnahmen aufweist, die jeweils eine entsprechende neue Spritze enthalten, wobei die Zähne (22) des Förderbandes mit einem ersten Paar Leerlaufzahnräder (23) sowie mit einem zweiten Paar Antriebszahnräder (24) in Eingriff sind, die mit betätigten Einrichtungen versehen sind, mit denen sie in einzelnen Abschnitten gedreht werden können, so daß nacheinander jede der Aufnahmen zur Entnahme der neuen Spritze darin auf die zweite Öffnung in dem Behälter ausgerichtet werden kann;
h) ein Blockiersystem, das die Drehung des Förderbandes blockiert und nur durch das Eindringen der Nadel der gebrauchten Spritze in die Maßbohrung gelöst werden kann;
i) eine Einrichtung, mit der das System zum Blockieren der Drehung des Förderbandes nach der Drehung des Förderbandes um einen Schritt wieder in eine Funktionsstellung gebracht werden kann, so daß die gebrauchte Spritze in ihre Ausgangsposition in ihrem Gehäuse an der Aufnahmerolle zurückkehrt,
**dadurch gekennzeichnet**, daß
die Drückeinrichtung die gebrauchte Spritze nur verschieben kann, wenn diese mit ihrer Nadel versehen ist, um so das Förderband durch die Nadel zu lösen und die Vorrichtung eine neue Spritze ausgeben zu lassen.

2. Vorrichtung nach Anspruch 1, wobei die Aufnahmerolle aus einem Zylinder besteht, der integral mit zahlreichen radialen Ansätzen (9) versehen ist, die in Umfangsrichtung gleichmäßig voneinander beabstandet sind, so daß zwischen jedem benachbarten Paar der Ansätze eines der Gehäuse entsteht.

3. Vorrichtung nach Anspruch 1 und 2, wobei die manuelle Betätigungseinrichtung, mit der die Aufnahmerolle in einzelnen Schritten gedreht wird, aus einer Kurbel (13) besteht, die sich außerhalb des Behälters befindet und mit der die Drehung der Aufnahmerolle in einzelnen Schritten gesteuert werden kann.

4. Vorrichtung nach einem der Ansprüche 1 bis 3, wobei die Drückeinrichtung durch einen Federhammer (17) gebildet wird, der durch das Gleiten über einen Vorsprung (16), der sich an der entsprechenden Seite jedes der Ansätze an der Aufnahmerolle befindet, gespannt wird, während der Drehung der letzteren nach vorn federt, wenn er nicht mehr mit dem Vorsprung in Kontakt ist, wenn sich die Aufnahmerolle dreht, so daß er heftig auf das Unterteil der gebrauchten Spritze aufschlägt, die sich vor ihm befindet.

5. Vorrichtung nach einem der vorangehenden Ansprüche, wobei die Betätigungseinrichtung zum Drehen des Paars Antriebszahnräder in einzelne Schritte aus einer Spiralfeder (29) besteht, die um eine Welle (28) gewickelt ist, die die Antriebszahnräder verbindet.

6. Vorrichtung nach einem der vorangehenden Ansprüche, wobei das System zum Blockieren der Drehung des Förderbandes einen ersten Hebel (30) umfaßt, der an seinem Mittelpunkt gelenkig gelagert ist und normalerweise mittels einer Spiralfeder (33) in einer vertikalen Stellung gehalten wird, wobei sich sein Oberteil der Maßbohrung gegenüber befindet, wobei die Maßbohrung dem Vorstehen der Nadel einer in der Aufnahmerolle aufgenommenen gebrauchten Spritze dient, und einen zweiten Hebel (31), der an einem Ende gelenkig an dem unteren Ende des ersten Hebels gelagert ist und sich horizontal erstreckt, bis er mit einem Zahn des Förderbandes in Kontakt kommt, um so Drehung desselben zu verhindern, wobei der Aufbau so ist, daß die Nadel der obenerwähnten gebrauchten spritze, wenn sie aus der Maßbohrung vorsteht, aufgrund der Aufschlagkraft, die durch die Wirkung des Federhammers entsteht, wenn die gebrauchte Spritze in ihr Gehäuse in der Aufnahmerolle gleitet, heftig auf den oberen Teil des ersten Hebels aufschlägt, so daß sich letzterer im Uhrzeigersinn um das Gelenk an seinem Mittelpunkt dreht und dadurch der zweite Hebel aus dem Eingriff mit dem Zahn des Förderbandes gezogen wird, das sich so ungehindert drehen kann.

7. Vorrichtung nach einem der vorangehenden Ansprüche, wobei die Einrichtung zum zurückführen des Systems, das die Drehung des Förderbandes blockiert, in eine Funktionsstellung und zum Zurückführen der gebrauchten Spritze in ihre Ausgangsstellung in dem Gehäuse an der Aufnahmerolle aus der Spiralfeder besteht, die dazu bestimmt ist, den ersten Hebei des Systems zum Blockieren der Drehung des Förderbandes normalerweise in einer vertikalen Stellung zu halten, wobei die Feder, die durch die Drehung des ersten Hebels im Uhrzeigersinn, die durch das Vorstehen der Nadel durch die Maßbohrung bewirkt wird, gespannt wird, bewirkt, daß sich der erste Hebel schnell entgegen dem Uhrzeigersinn zurückdreht, und ihn so in die vertikale Stellung zurückführt, wodurch sich der zweite Hebel, der mit einem der Zähne des Förderbandes in Eingriff ist, bewegt, wobei der erste Hebel während der Drehung entgegen dem Uhrzeigersinn eine Drückwirkung auf die Nadel ausübt, die aus der Maßbohrung vorsteht, so daß die gebrauchte Spritze, die zu der Nadel gehört, zurückgleitet, bis sie die Position erreicht, die sie ursprünglich in ihrem Gehäuse an der Aufnahmerolle einnahm, bevor sie dem Druck des Federhammers ausgesetzt war.

8. Vorrichtung nach einem der vorangehenden Ansprüche, wobei die neuen Spritzen in den Aufnahmen an dem Förderband vollständig in einer Phiole mit physiologischer Lösung oder destilliertem Wasser verpackt sind.

## Revendications

1. Appareil pour le recueil de seringues usagées (10) et la distribution conditionelle de nouvelles seringues (21), comprenant:
a) un réservoir en forme de placard (1) avec au moins une porte munie d'une serrure de sécurité donnant l'accès à l'intérieur, ce réservoir étant séparé par une paroi (2) parallèle aux parois latérales en un premier compartiment (3) et en un second compartiment (4) destinés respectivement au recueil de seringues usagées et à la distribution de nouvelles seringues;
b) une première et seconde ouvertures dans le réservoir respectivement pour l'introduction de seringues usagées dans le premier compartiment et la distribution de nouvelles seringues à partir du second compartiment;
c) un rouleau de réception (7) logé de façon rotative à l'intérieur du premier compartiment sur l'arbre (11), ce rouleau étant divisé circonférentiellement en un certain nombre de logements s'étendant axialement, chacun de ces logements étant apte à recevoir une seringue usagée introduite par la première ouverture dans le réservoir, le rouleau étant constitué d'un cylindre (8) fixé sur l'arbre, une première extrémité de l'arbre étant montée de façon rotative sur le réservoir tandis que l'autre extrémité est montée de façon rotative sur une plaque (12);
d) des moyens d'actionnement manuel (14) pour faire tourner le rouleau de réception par pas individuels de façon à amener chacun des logements successivement en alignement sur la première ouverture;
e) des moyens de poussée (17) coopérant avec des moyens d'actionnement pour faire coulisser les seringues usagées introduites dans l'un des logements sur le rouleau de réception et en alignement sur un alésage étalonné placé sur l'extrémité d'une encoche (15) ménagée dans la plaque;
f) un réservoir (18) situé au-dessous du rouleau de réception pour recueillir les seringues usagées qui tombent par gravité aux pas individuels à mesure que le rouleau récepteur effectue une rotation;
g) une bande transporteuse circulaire crantée (19) située dans le second compartiment, présentant sur son périmètre une succession de réceptacles dont chacun contient une nouvelle seringue, les crans (22) de la bande transporteuse coopérant avec une première paire de roues dentées non-entraînées (23), une seconde paire de roues dentées entrainées (24) munies de moyens actionnés capables de les faire tourner dans des sections individuelles dé façon à amener en succession chacun des réceptacles en correspondance avec la seconde ouverture dans le réservoir pour le prélèvement de la nouvelle seringue;
h) un système de blocage pour bloquer la rotation de la bande transporteuse pouvant être débloquée uniquement par la saillie de l'aiguille de la seringue usagée à travers l'alésage étalonné;
i) des moyens pour amener le système de blocage pour la rotation de la bande transporteuse en retour sur une position opérante, suivie de la rotation d'un pas de la bande transporteuse et du retour de la seringue usagée sur sa position de départ dans le logement sur la rouleau de réception,
caractérisé par le fait que
les moyens de poussée ne peuvent faire coulisser la seringue usagée que si elle est munie de son aiguille, provoquant ainsi le déblocage de la bande transporteuse par l'aiguille et autorisant l'appareil à libérer une nouvelle seringue.

2. Appareil selon la revendication 1, dans lequel le rouleau de réception est constitué d'un cylindre solidaire de nombreuses languettes radiales (9), circonférentiellement equidistantes entre elles de façon à former l'un des logements entre chaque paire contiguë de languettes.

3. Appareil selon les revendications 1 ou 2, par lequel les moyens d'actionnement manuel pour faire tourner le rouleau de réception par pas individuels sont constitués par une manivelle (13) située à l'extérieur de réservoir, permettant de commander la rotation par pas individuels du rouleau de réception.

4. Appareil selon l'une quelconque des revendications 1 à 3, dans lequel les moyens de poussée sont constitués par un marteau à ressort (17) qui, précontraint lors du passage au-dessus d'une saillie (16), située sur le côté correspondant de chacune des languettes du rouleau de réception, pendant la rotation de ce dernier, jaillit sur l'avant lorsqu'il n'est plus engagé par la saillie lorsque le rouleau de réception est en rotation de façon à heurter violemment la base de la seringue usagée, située en correspondance avec celui-ci.

5. Appareil selon l'une quelconque des revendications précédentes, dans lequel des moyens d'actionnement pour la rotation par pas individuels de la paire de roues dentées entraînées sont formés par un ressort hélicoïdal enroulé sur un arbre (28) reliant les roues dentées entraînées.

6. Appareil selon l'une quelconque des revendications précédentes, dans lequel le système de blocage de la rotation de la bande transporteuse comprend un premier levier (30) articulé sur son point médian et normalement maintenu, au moyen du ressort hélicoïdal (33), dans une position verticale avec sa partie supérieure opposée à l'alésage étalonné, cet alésage étalonné étant destiné à le saillie de l'aiguille d'une seringue usagée recueillie dans le rouleau récepteur, et un second levier (31) avec une extrémité articulée sur l'extrémité inférieure du premier levier et qui s'étend horizontalement jusqu'à l'engagement avec un cran de la bande transporteuse de façon à empêcher sa rotation, l'agencement étant tel que lorsque l'aiguille de la seringue usagée précitée fait saillie depuis l'alésage étalonné, elle vient violemment heurter la partie supérieure du premier levier, grâce à la force d'impact obtenue lorsque la seringue usagée coulisse dans son logement dans le rouleau récepteur suivant l'action du marteau à ressort, provoquant la rotation dans le sens horaire de ce dernier et le retour de l'articulation sur son point médian, avec la sortie correspondante du second levier de l'engagement du cran de la bande trans-porteuse qui put alors tourner librement.

7. Appareil selon l'une quelconque des revendications précédentes, dans lequel les moyens pour ramener ce système pour le blocage de rotation de la bande transporteuse dans une position opérante et pour le retour de la seringue usagée dans sa position de départ à l'intérieur du logement sur le rouleau récepteur sont constitués par le ressort hélicoïdal, destiné normalement à maintenir le premier levier du système pour bloquer la rotation de la bande transporteuse dans une position verticale, le ressort, sollicité par la rotation horaire du premier levier déterminé par la saillie de l'aiguille à travers l'alésage étalonné, provoque la contre-rotation du premier levier rapidement dans une direction anti-horaire le ramenant dans la position verticale avec le mouvement correspondant du second levier en engagement sur un cran de le bande transporteuse, le premier levier exerçant pendant la rotation anti-horaire une action de poussée sur l'aiguille faisant saillie à partir de l'alésage étalonné de façon à faire coulisser en arrière la seringue usagée, associée à cette aiguille jusqu'à la position qu'elle avait à l'origine à l'intérieur de son logement sur le rouleau récepteur avant de subir la force de poussée du marteau à ressort.

8. Appareil selon l'une quelconque des revendications précédentes, dans lequel les nouvelles seringues à l'intérieur des réceptacles sur la bande transporteuse sont entièrement conditionnées avec un flacon de solution physiologique ou d'eau distillée.
